# EUROPEAN PATENT APPLICATION

(11) **EP 4 534 552 A1**
(43) Date of publication of application: **09.04.2025**
(21) Application number: 23461657.1
(22) Date of filing: 04.10.2023
(51) Int. Cl.: C07K 14/47, C07K 1/14, C12N 5/07, C12N 15/866

(54) **HIGHLY EFFICIENT METHOD FOR OBTAINING AND PURIFICATION OF HUMAN ELP 123 COMPLEX**

(71) Applicant: UNIWERSYTET JAGIELLONSKI, 31-007 Kraków (PL)
(72) Inventor: Ting-Yu, Lin, Bishop Auckland, DL13 5HQ (GB); Glatt, Sebastian, 30-073 Kraków (PL)
(74) Representative: Witek, Rafal

(57) **Abstract**

In subject application a highly efficient method for obtaining and purification of functional human Elp123 complex has been disclosed.

## Description

Subject of the invention is highly efficient method for obtaining and purification of human Elp123 complex.

### State of the art

Proteins are the work horse in cells and dysfunctions in them lead to diseases. Understanding how the faulty proteins causes dysregulation is crucial to explain the disease formation. Therefore, characterizations of protein features, including structure (complex assembly and ligand coordination) and activities, are the key to fill the knowledge gap. Proteins can be obtained from cells directly or recombinantly produced. The latter one becomes a common practice due to higher yield which is important for down stream applications. Producing recombinant proteins can be achieved by performing the expression in various systems, such as bacteria, yeast or insect cells.

Elongator complex (Elp), a multi-subunit complex (Elp1-6), is responsible for the modification at uridine 34 in some tRNAs. Elp-mediated reaction requires ligands, including acetyl-CoAand SAM, which are bound by the catalytic subunit Elp3. The modification is crucial to fine-tune translation machinery and the dysregulation of the Elp-mediated reaction has been linked to neurodegenerative diseases and cancers. Each subunits can be produced via bacterial system, but the eukaryotic Elp3 is not soluble despite several attempts using bacterial or yeast systems, which makes it difficult to dissect Elp3 roles in the relations to other subunits with the regards in understanding human Elp activity.

Kojic M. et. al. disclose in Journal of Human Genetics (2023) 68:445-453; https://doi.orq/10.1038/s10038-023-01135-3 a method for obtaining of Elp123 complex based on using of SuperSf9-3 cells transformed with pFastBac1 HTa including codon-optimized open reading frames of human Elp1 (095163), Elp2 (Q61A86) and Elp3 (Q9H9T3).

Moreover, Gaik M et al. describes in Functional divergence of the two Elongator subcomplexes during neurodevelopment. EMBO Mol Med.2022;14:e15608. https://doi.org/10.15252/emmm.202115608 construct for production of the ELP456 complex from Homo sapiens in insect cells.

### Technical problem

The aim of the invention is to propose a highly efficient method for obtaining the functional human Elp123 complex.

Surprisingly, this problem is solved by the present invention, the essence of which is defined in the appended claims.

### Detailed description of the invention

Performing the standard purification method to purify Elp123 complex results in low yield which is not feasible for downstream application. Inventors optimized protein purification method to obtain around 300 microgram of protein. The optimization includes i) the lysis procedure using a microfluidizer instead of conventional sonication, ii) the addition of benzonase instead of adding DNase alone, iii) optional step of heparin column to remove excess of nucleic acid contamination, iv) size exclusion purification using a s6 Superose column within the same day.

The complex obtained according to invention carries mutations and allows to characterize their effects on enzyme activity, including tRNA substrate binding, acetyl-CoA hydrolysis and complex integrity. The structure of obtained human Elp123 complex have been analyzed using the single-particle cryoEM. As the Elp complex also contains the ligand, the ligand binding features can be determined. The information is useful for future small drug screening to compete with the ligand binding for potential therapeutic applications.

**Example 1.** Highly efficient method for obtaining and purification of human Elp123 complex.

### Construct production

Codon-optimized open reading frames of Elp1 (095163), Elp2 (Q61A86) and Elp3 (Q9H9T3) were cloned into pFastBac1 HTa. Elp3 was cloned with an additional in-frame Twin-strep-Tag at its 3' end. The Elp123 construct was generated using Gibson assembly by amplifying all three genes in PCR with primers adding specific overhangs on 5' and 3' sides of each expression cassette, allowing to determine the specific order of the ORFs. Mutations in Elp123 were introduced by QuikChange mutagenesis.

### QuikChange Mutagenesis

DNA template (50 ng) was mixed with the forward primer (1 µM) or reverse primer (1 µM). The two mixtures were then further mixed with Phusion polymerase (0.5 U), dNTP, 1x PCR reaction buffer, DTT (10 µM). After mixing, the two vials were subject to standard PCR procedure for 30 cycles with the following settings: denaturing at 98 °C for 10 sec, primer annealing at 45 °C for 10 sec and extension at 72 °C for 4 minutes. Once the PCR finished, the two mixtures were treated with CIP at 37 °C for 30 minutes to remove the template. The two mixtures were then mixed and subjected to annealing procedure with the following settings: 85 °C for 20 sec, 80 °C for 20 sec, 75 °C for 20 sec, 70 °C for 20 sec, 65 °C for 20 sec, 60 °C for 20 sec, 55 °C for 20 sec, 50 °C for 20 sec, 45 °C for 20 sec, 40 °C for 20 sec, and 35 °C for 5 minutes. The mixture was then transformed into the DH5 competent cells and the DNA was extracted from the culture for the subsequent sequencing to confirm the mutation.

### Protein purification

For Elp123 protein expression, SuperSf9-3 cells were infected with multiplicity of infection (MOI) = 1 with baculovirus vector with SEQ ID Nr 1 and grown for 3 days at 27 °C on a shaking platform in culture medium ESF 921 Insect Cell Media (serum-free, protein-free insect cell culture medium). Subsequently, insect cells (from 3 L cultures) were lysed in Lysis Buffer (for Elp123: 50 mM HEPES pH 7.5, 100 mM NaCl, 2 mM DTT, 5% glycerol, DNase I, protease inhibitors; by 3 cycles of freezing and thawing in liquid nitrogen and pass-through a microfluidizer to homogenize cells without overheating the lysates. Elp123 were first purified using a StrepTrap HP 5 ml column (GE Healthcare). The lysates were applied onto the column and circulate at least 2 times to increase the yield. The proteins were eluted in Strep Elution Buffer (50 mM HEPES, 100 mM NaCl, 1 mM DTT, 5% glycerol, 10 mM d-desthiobiotin, pH 7.5), followed by affinity chromatography on HiTrap Heparin HP 5 ml column (GE Healthcare). The target protein was again eluted in a gradient of Heparin Elution Buffer (50 mM HEPES, 1 M KCI, 1 mM DTT, pH 7.5). Finally, eluates were run on Superose 6 Increase 10/300 GL column (GE Healthcare) in 20 mM HEPES pH 7.5, 100 mM NaCl, 5 mM DTT. Selected fractions were pooled and concentrated with on an Amicon Ultra-0.5 (100 kDa cut-off) concentrator. The purification procedure should be finished within one day to ensure the protein is active. Aliquots were frozen in liquid nitrogen and stored at -80 °C for further use.

The process efficiency was 200-300 µg of Elp123 proteins from 3 L cell culture.

### Example 2. Comparative.

Human Elp123 complex has been obtained with the known method which has been described in publication "A novel ELP1 mutation impairs the function of the Elongator complex and causes a severe neurodevelopmental phenotype", Marija Kojic et al. Journal of Human Genetics (2023) 68:445-453; https://doi.org/10.1038/s10038-023-01135- 3.

The process efficiency was >80 µg from 3-6 L cell culture. It was 3-6 times lower than obtained in method according to the invention.

## Claims

1. Method for obtaining human Elp123 complex, **characterized in that**:
a) insect cells transformed with baculovirus vector with SEQ ID Nr 1 are grown for 3 days at 27°C in fluid culture medium,
b) said insect cells are lysed in lysis buffer comprising 50 mM HEPES pH 7.5, 100 mM NaCl, 2 mM DTT, 5% glycerol, DNase I, protease inhibitors and benzonase (1 U)) by 3 cycles of freezing and thawing in liquid nitrogen and pass-through a high pressure microfluidization homogenizer to homogenize cells without overheating the lysates,
c) said lysates are applied onto the affinity column packed with medium having affinity to streptactin, 6% Highly Cross-linked Agarose, circulated at least 2 times and fraction comprising Elp123 complex is eluted in elution buffer comprising 50 mM HEPES, 100 mM NaCl, 1 mM DTT, 5% glycerol, 10 mM d-desthiobiotin, pH 7.5),
d) obtained fraction comprising Elp123 complex is applied onto the affinity column packed with medium having affinity to heparin and fraction comprising Elp123 complex is eluted in a gradient of elution buffer comprising 50 mM HEPES, 1 M KCI, 1 mM DTT, pH 7.5),
e) obtained fraction comprising Elp123 complex are run on size exlusion column packed with polymeric cross-linked agarose and eluted in elution buffer comprising in 20 mM HEPES pH 7.5, 100 mM NaCl, 5 mM DTT,
f) Selected fractions comprising Elp123 complex are pooled and concentrated on an membrane having 100 kDa cut-off.

2. Method according to claim 1, wherein in step a) cell line derived from the Spodoptera frugiperda (Sf9) in used.

3. Method according to claim 1, wherein in step b) said cells are homogenized in the cold room (4 °C) and with pressure of 15000 psi.

4. Method according to claim 1, wherein in step c) said column is packed with the agarose beads that bind to Strep-tagged protein of interest.

5. Method according to claim 1, wherein in step d) said column is packed with heparin resin that binds to nucleic acid-binding proteins which allow to remove the nucleic acid from the sample.

6. Method according to claim 1, wherein in step e) said column is packed with agarose-based matrix for size exclusion chromatography to separate the protein complex of interest from other contaminants.

7. Method according to claim 1, wherein in step f) Centrifugal Filter Unit [100 kDa cut-off which retains the complex (860 kDa)) is used.

8. Baculovirus vector having SEQ ID Nr 1.
